Europäisches Patentamt

European Patent Office

Office européen des brevets

(11) Publication number : **0 393 511 B1**

# (12) EUROPEAN PATENT SPECIFICATION

(45) Date of publication of patent specification :
**11.11.92 Bulletin 92/46**

(51) Int. Cl.$^5$ : **C09D 5/00,** C08J 3/12,
// C08L83/04, C08K5/54

(21) Application number : **90107027.6**

(22) Date of filing : **12.04.90**

(54) **Anti-microbial silicone rubber particles.**

(30) Priority : **17.04.89 JP 96849/89**

(43) Date of publication of application :
**24.10.90 Bulletin 90/43**

(45) Publication of the grant of the patent :
**11.11.92 Bulletin 92/46**

(84) Designated Contracting States :
**BE DE FR GB**

(56) References cited :
GB-A- 1 593 217
US-A- 4 602 959
US-A- 4 835 019

(73) Proprietor : **Dow Corning Toray Silicone
Company Ltd.
3-16, Nihombashi-muromachi 2-chome,
Chuo-ku
Tokyo 103 (JP)**

(72) Inventor : **Yoshida, Keiji
6,1-chome, Yushudai Nishi
Ichihara-shi, Chiba (JP)**
Inventor : **Hamada, Mitsuo
5-17, 3-chome, Ohkubo
Kisarazu-shi, Chiba (JP)**

(74) Representative : **Spott, Gottfried, Dr.
Patentanwälte Spott und Puschmann
Sendlinger-Tor-Platz 11
W-8000 München 2 (DE)**

## Description

The present invention relates to an anti-microbial material. More particularly, this invention relates to an antimicrobial material in the form of particles of a cured silicone rubber. In addition to imparting anti-microbial properties the particles also desirably modify the physical properties of a variety of products, including organic resins, rubbers, paints and cosmetics.

Particulate forms of cured silicone rubber have recently entered into use as internal stress-relaxing agents, impact-strength modifiers, and lubricity modifiers for organic resins. These silicone rubber particles have also been evaluated as surface modifiers for various types of paints.

The use of silicone materials in a paint is taught in Japanese published patent application (Kokai) Number 62-25180 [25,180/87]. In accordance with the teaching of this application beads of a silicone material are blended into a room temperature-curable or thermosetting paint. The cured paint has a rough or uneven surface that is adhesive-repellent, and is suitable for resisting or preventing adhesion to the painted surface of gummed or adhesive paper in the form of posters and labels.

Particulate silicone materials have also been evaluated as replacements for the bodying pigments, coloring pigments, oils, activators, nylon powders, cellulose powders, and other ingredients used in cosmetics, and particularly in powders intended for use as cosmetics.

A shortcoming of the silicone material presently used as additives in paint and cosmetics is their inability to inhibit the growth of naturally occurring bacteria, mold, and other organisms. This has limited the use of silicone materials, particularly powder forms of cured silicone rubber, in those applications where a resistance to the development of bacterial and/or mold is required.

Particles coated with anti-microbial organosilicon compounds are described in British patent No. 1,433,303. The antimicrobial compounds are silanes containing a quaternized amine group and polyorganosiloxanes containing the quaternized nitrogen atom of a pyridine ring bonded to silicon. The antimicrobial compound is coated on the surface of a material in particulate form, to which it becomes strongly bonded. The coated particles are then incorporated into a polymeric matrix, which can subsequently be fabricated into a shaped article or applied as a coating.

The particulate material that is coated with the anti-microbial compound can be organic or inorganic, and includes metals, metal oxides and carbonates, siliceous materials, cellulosic materials, resins and plastics. Elastomeric materials are conspicuous by their absence.

One objective of the present invention is to impart antimicrobial properties to cured silicone rubbers in powder form without adversely affecting the ability of the silicone rubber to function as an additive or modifier for both liquid and solid materials.

The present inventors found that anti-microbial organosilicon compounds can be blended into curable silicone rubber composition without adversely affecting either the ability of the composition to be cured in particulate form or the ability of the resultant particles to effectively modify the physical properties, such as lubricity and impact resistance, of organic or inorganic matrices into which the particles are incorporated as additives.

The present invention relates to an anti-microbial composition comprising particles of a cured silicone rubber exhibiting an average diameter of from 0.1 to 500 micrometers and containing from 0.1 to 30 weight percent of an organosilicon quaternary ammonium salt as the anti-microbial agent.

Any of the known organosilicon quaternary ammonium salts exhibiting antimicrobial properties can be used as the antimicrobial agent in the present cured silicone rubber compositions. Representative classes of quaternary ammonium salts are disclosed in British patent No. 1,433,303, discussed in the prior art section of this specification. No particular restriction applies to the quaternary ammonium-substituted organosilicon compounds that can be used in combination with a curable organosilicone rubber composition to prepare the present particles.

Preferred quaternary ammonium-substituted organosilicon compounds include octadecyldimethyl[3-(trimethoxysilyl)-propyl]ammonium chloride and its partial hydrolyzate.

The concentration of the anti-microbial organosilicon compound is typically from 0.1 to 30 weight percent, preferably from 1 to 20 weight percent, based on the total weight of the present silicone rubber particles. At concentrations below about 0.1 weight percent the desired anti-microbial effect is not evident when the cured silicone rubber particles are added to a matrix material such as an organic resin or plastic. When the concentration of anti-microbial organosilicon compound exceeds about 30 weight percent, based on the weight of the rubber particles, the curing reaction used to prepare the particles is strongly inhibited.

To function effectively as an additive for modifying the physical properties of the matrix into which the particles will be ultimately incorporated, the average diameter of the particles should be from 0.1 to 500 micrometers. An average particle diameter below 0.1 micrometer results in a reduced modification of the physical properties, while miscibility with various matrix materials into which the present particles are incorporated as additives typically cannot be achieved when the average diameter of the particles exceeds 500 micrometers.

The curable silicone rubber composition used to prepare the particles of this invention can be any of the curable liquid compositions described in the art. The only requirement is that the composition be dispersible in water.

Examples of suitable curable compositions include organoperoxide-curing silicone rubbers, in which at least one vinyl-containing diorganopolysiloxane is cured by the heat-induced decomposition of an organoperoxide; addition reaction-curing silicone rubbers which cure by the reaction between the silicon-bonded hydrogen atoms of an organohydrogenpolysiloxane and the alkenyl radicals, such as vinyl, of a diorganopolysiloxane in the presence of a platinum-type compound catalyst; condensation reaction-curing silicone rubbers which cure either by the reaction between at least one hydroxyl-terminated diorganopolysiloxane and the silicon-bonded hydrogen atoms of at least one organohydrogenpolysiloxane in the presence of an organotin compound, or by the reaction between at least one hydroxyl-terminated diorganopolysiloxane and at least one organosilane containing hydrolyzable groups in the presence of an organotin compound or a titanate ester.

Among these types of curable compositions addition reaction-curing and condensation reaction-curing compositions are preferred, based on their ease of preparation and handling. Most preferably the silicon-bonded hydrocarbon radicals other than alkenyl that are present in said diorganopolysiloxane and said organohydrogenpolysiloxane are methyl.

A variety of methods are available for converting a curable liquid silicone rubber composition to cured particles of this invention that incorporate the antimicrobial compounds of this invention. Some of the preferred methods can be summarized as follows:

(1) A liquid silicone rubber composition containing a dispersed organosilicon quaternary ammonium salt is first prepared by blending an organosilicon quaternary ammonium salt into a liquid addition reaction-curing silicone rubber composition consisting essentially of an organopolysiloxane having in each molecule at least two alkenyl radicals (typically vinyl), an organohydrogenpolysiloxane having in each molecule at least two silicon-bonded hydrogen atoms, and a metal from the platinum group of the periodic table or a compound of one of these metals as the curing catalyst. This composition is then introduced with stirring into water alone or into surfactant-containing water to disperse the curable composition in the form of liquid microparticles. To avoid premature curing the temperature of the resultant aqueous dispersion should be maintained below about 25°C. The dispersed microparticles are then cured either by spraying the aqueous dispersion into heated air or by adding the aqueous dispersion with stirring to water heated to at least 25 degrees Centigrade.

(2) A liquid silicone rubber composition containing a organosilicon quaternary ammonium salt is prepared by blending an organosilicon quaternary ammonium salt into a liquid condensation reaction-curing silicone rubber composition prepared from at lest one organopolysiloxane having at least 2 terminal hydroxyl groups, at least one organohydrogenpolysiloxane having in each molecule at least 2 silicon-bonded hydrogen atoms, and an organotin catalyst. This curable composition is then introduced with stirring into water alone or into surfactant-containing water, and the temperature is maintained below about 25°C to form an aqueous dispersion of the curable composition. The resultant aqueous dispersion is then cured by allowing the water-based dispersion to stand as such for an extended period, by heating it, or by spraying it into heated air using a spray drier or similar device.

In addition to the antimicrobial organosilicon compound the curable liquid silicone rubber compositions used to prepare the present cured rubber particles can contain those additives as known in the art. These additives include but are not limited to fillers such as silica, hydrolyzable organosilanes and pigments.

When the antimicrobial silicone particles of this invention are used as a modifying additive for other materials, a particle concentration of at least 1.0 weight percent is typically required to achieve an effective level of anti-bacterial and anti-mold activity.

The following are examples of liquid and solid matrices into which the anti-microbial silicone rubber particles can be incorporated: solid lubricants, water repellents, release agents, tack inhibitors, greases, oils, cements, plasters, paints, casting materials, molding materials, films, fibers, cosmetics, and medicaments. Polymers into which the cured silicone rubber particles can be incorporated to impart antimicrobial properties in addition to modifying physical properties include but are not limited to silicone rubbers, natural rubbers, polychloroprene rubbers, polybutadiene rubbers, styrene/butadiene rubbers, ethylene/propylene rubbers, polyisoprene rubbers, polyisobutene rubbers, polyacrylate ester rubbers, polyurethane rubbers, butadiene-acrylonitrile copolymer rubbers, polyester rubbers, polysulfide rubbers, fluororubbers, copolymers and mixtures derived from the preceding rubbers. Resins into which the present anti-microbial cured rubber particles can be incorporated are exemplified by the various thermoplastic and thermosetting resins and by resins which are cured by high-energy radiation (UV, gamma, electron beam). Examples of suitable resins include but are not limited to polyamides, including nylon 5, nylon 6, nylon 7, nylon 8, nylon 9, nylon 10, nylon 11, nylon 12, nylon 66, and aromatic polyamides such as Kevlar®;

saturated polyesters such as polyethylene terephthalate, polybutylene terephthalate, poly-hydrogenated xylylene terephthalate, polycaprolactone, and polypivalolactone; polycarbonate; acrylonitirile/butadiene/styrene (ABS) terpolymers; AS copolymers; polystyrene; polyethylene; polypropylene; polybutadiene; polyvinyl chloride; polyvinylidene chloride; polyacrylonitrile; polyvinyl alcohol; polyvinyl acetate; polyvinyl butyral; polymethyl methacrylate; fluororesins; other polyolefin resins; polyethers such as polyethylene glycol, polypropylene glycol, polytetrahydrofuran, Penton®, polyphenylene oxide, and polyacetals; phenolic resins; polyurethane resins; acrylic resins; urea resins; unsaturated polyesters; melamine resins; phthalic acid resins; BT resins; polyimide resins; silicone resins; celluloid; acetylcelluloses; epoxy acrylates; polyacrylates; and epoxy resins. Block and random copolymers derived from two or more of the preceding resins as well as blends of these resins are also suitable matrices for the present cured antimicrobial silicone rubber particles.

## Examples

The present invention is explained in greater detail by means of the following illustrative examples, which should not be interpreted as limiting the scope of the invention defined in the accompanying claims. Unless otherwise specified all parts and percentages in the examples are by weight, and viscosity values were measured at 25 degrees Centigrade.

The performance of samples containing the cured rubber particles of this invention as inhibitors for the growth of bacteria and mold was indicated by the presence or absence of a zone of growth inhibition in a culture medium using the procedure described in the "Antimicrobial (Mold) Test Method" of JIS Z 2911-1981.

In accordance with this procedure 1.0 cc of the appropriate liquid inoculate was smeared onto a standard agar culture medium for bacteria or onto a potato dextrose agar culture medium for mold, and the test specimen was placed on the flat agar slab. The presence or absence of growth inhibition was determined by visual observation after cultivation of the inoculate for 48 hours at 35 degrees Centigrade in the case of bacteria or for 7 days at 25 degrees Centigrade in the case of mold.

## Example 1

A liquid curable silicone rubber composition was prepared by mixing the following ingredients to homogeneity: 100 parts of a hydroxyl-terminated dimethylpolysiloxane exhibiting a viscosity of 1,000 centistokes ($1 \times 10^{-3}$ m²/sec.) and a hydroxyl group content of 1.3 weight %, 10 parts trimethylsiloxy-terminated methylhydrogenpolysiloxane exhibiting a viscosity of 10 ($1 \times 10^{-5}$ m²/sec.) centistokes and a silicon-bonded hydrogen content of 1.5 weight %, 10 parts of octadecyldimethyl[3-(trimethoxysilyl)propyl]ammonium chloride, and 1.0 part of dibutyltin dioctoate. 100 Parts of the resultant composition was then introduced into a colloid mill (manufactured by the Manton-Gaulin Company), followed immediately by 1,000 parts of ion-exchanged water and 5 parts of a nonionic surfactant (the ethylene oxide adduct of trimethylnonanol). Milling of the resultant mixture at 800 rpm using a 0.1 mm gap yielded an aqueous dispersion of a curable silicone rubber composition of this invention.

Using a spray dryer manufactured by Ashizawa-Niro Atomizer Limited this aqueous dispersion was sprayed into a hot air dryer using an inlet temperature of 300 degrees Centigrade and an outlet temperature of 100 degrees Centigrade. The resultant cured antimicrobial silicone rubber particles corresponded to an embodiment of the present invention.

Observation of the cured silicone rubber particles using electron microscopy revealed them to be spherical with an average particle diameter of 40 micrometers.

Ten grams of these particles were dispersed in 50 g toluene, and the resultant dispersion was blended into 100 g of a polyurethane resin paint available as "Rethane White" from the Kansai Paint Company, Limited. Twenty grams of a curing agent for the polyurethane resin was blended into the paint and mixed to homogeneity to yield a curable paint composition. This composition was applied to a sheet of Teflon®. Following a 24 hour exposure under ambient conditions the surface of the paint film had a matte finish resembling that of a pear skin.

When a portion of this film was used as a test specimen for evaluation of anti-microbial activity, an area of growth inhibition surrounding the test sample was observed in both the bacteria and mold cultures.

## Example 2

A curable liquid silicone rubber composition was prepared by adding the following ingredients with stirring to 100 parts of a dimethylvinylsiloxy-terminated dimethylpolysiloxane exhibiting a viscosity of 500 centistokes ($5 \times 10_{-4}$ m²/sec.) and a vinyl radical content of 0.5 weight percent: 5 parts octadecyldimethyl[3-(trimethoxysilyl)propyl]ammonium chloride, 6 parts trimethylsiloxy-terminated methylhydrogenpolysiloxane exhibiting a viscosity of 10 centistokes and a silicon-bonded hydrogen content of 1.5 weight percent, and 0.3 parts isopropanolic chloroplatinic acid solution exhibiting a platinum content of 3 weight percent. 100 Parts of this curable composition was introduced into a colloid mill, followed immediately by 100 parts of ion-exchanged water and 2.5 parts of a surfactant identified as polyoxyethylene lauryl ether. An aqueous dispersion of a curable silicone rubber composition of this invention was obtained by milling the mixture at 1,400 rpm using a 0.1 mm gap.

The resultant aqueous dispersion was cured by passing it through a spray drier using the conditions described in Example 1. The average particle size of the resultant cured silicone rubber particles was 10 micrometers.

A cosmetic composition in the form of a powder was prepared by mixing the following ingredients to homogeneity: 5 parts of the cured silicone rubber particles prepared as described in the preceding section of this example, 50 parts mica, 10 parts kaolin, 2 parts squalane, and 10 parts of a liquid dimethylpolysiloxane. The resultant cosmetic exhibited an excellent slipperiness and smoothness. When this cosmetic was used as the test specimen in the evaluation for anti-bacterial and anti-mold performance described in a preceding section of this specification, zones of growth inhibition were present in both the bacteria and mold cultures.

Example 3

5 Parts of the cured silicone rubber powder prepared as described in example 1 of this specification were added to 100 parts of a butadiene-acrylonitrile copolymer rubber. The composition was then foamed and vulcanized to produce a sponge rubber with an excellent lubricity. When the anti-microbial activity of the sponge rubber was evaluated using the test described in a preceding section of this specification, zones of growth inhibition surrounding the test sample were observed in both the bacteria and mold cultures.

**Claims**

1. Particulate anti-microbial composition comprising particles exhibiting an average diameter of from 0.1 to 500 micrometers comprising a cured silicon rubber and from 0.1 to 30 weight percent of an organosilicon quaternary ammonium salt as an anti-microbial agent.

2. A composition according to claim 1 where said particles are prepared by heating an aqueous dispersion of a liquid silicone rubber composition curable by a platinum-catalyzed hydrosilation reaction or by the reaction between a hydroxyl-terminated diorganopolysiloxane and a curing agent selected from the group consisting of organohydrogenpolysiloxanes and hydrolyzable organosilanes.

3. A composition according to claim 2 where said liquid silicone rubber composition comprises 1) a diorganopolysiloxane containing at least two alkenyl radicals per molecule, an organohydrogenpolysiloxane containing at least two silicon-bonded hydrogen atoms per molecule and said hydrosilation catalyst, or 2) a hydroxyl-terminated diorganopolysiloxane, said organohydrogenpolysiloxane and an organotin compound as the curing catalyst.

4. A composition according to claim 3 where the silicon-bonded hydrocarbon radicals other than said alkenyl radicals present in said diorganopolysiloxane and said organohydrogenpolysiloxane are methyl.

5. A composition according to claim 1 where said particles are dispersed in a paint.

6. A composition according to claim 1 where said particles are dispersed in a rubber.

7. An composition according to claim 1 where said particles are dispersed in a powdered cosmetic.

**Patentansprüche**

1. Teilchenförmige antimikrobielle Zusammensetzung umfassend Teilchen aus einem gehärteten Siliconkautschuk mit einem durchschnittlichen Durchmesser von 0,1 bis 500 μm, die 0,1 bis 30 Gew.-% eines quaternären Organosiliciumammoniumsalzes als antimikrobielles Mittel enthalten.

2. Zusammensetzung nach Anspruch 1, worin die Teilchen hergestellt werden, indem eine wäßrige Dispersion einer flüssigen Siliconkautschukzusammensetzung, die durch eine platinkatalysierte Hydrosilylierungsreaktion härtbar ist, erhitzt wird oder durch Reaktion eines Diorganopolysiloxans mit Hydroxylendgruppen mit einem Härtungsmittel ausgewählt aus der Gruppe bestehend aus Organohydrogenpolysiloxanen und hydrolysierbaren Organosilanen.

3. Zusammensetzung nach Anspruch 2, worin die flüssige Siliconkautschukzusammensetzung umfaßt 1) ein Diorganopolysiloxan, das mindestens zwei Alkenylreste pro Molekül enthält, ein Organohydrogenpolysiloxan, das mindestens zwei siliciumgebundene Wasserstoffatome pro Molekül enthält und den Hydrosilylierungskatalysator oder 2) ein Diorganopolysiloxan mit Hydroxylendgruppen, das Organohydrogenpolysiloxan und eine Organozinnverbindung als Härtungskatalysator.

**4.** Zusammensetzung nach Anspruch 3, worin die silicongebundenen Kohlenwasserstoffreste, die außer den Alkenylresten in dem Diorganopolysiloxan und dem Organohydrogenpolysiloxan vorhanden sind, Methylreste sind.

**5.** Zusammensetzung nach Anspruch 1, worin die Teilchen in einer Farbe dispergiert sind.

**6.** Zusammensetzung nach Anspruch 1, worin die Teilchen in einem Kautschuk dispergiert sind.

**7.** Zusammensetzung nach Anspruch 1, worin die Teilchen in einem pulverförmigen Kosmetikum dispergiert sind.

**Revendications**

**1.** Composition particulaire antimicrobienne comprenant des particules présentant un diamètre moyen allant de 0,1 à 500 micromètres, comprenant un caoutchouc de silicone durci et de 0,1 à 30 pourcent en poids d'un sel d'ammonium quaternaire organo-silicié comme agent antimicrobien.

**2.** Composition selon la revendication 1, dans laquelle lesdites particules sont préparées en chauffant une dispersion aqueuse d'une composition de caoutchouc de silicone liquide durcissable par une réaction d'hydrosilation catalysée par le platine ou par la réaction entre un diorganopolysiloxane terminalement hydroxylé et un agent de durcissement choisi dans le groupe constitué des organohydrogéno-polysiloxanes et des organosilanes hydrolysables.

**3.** Composition selon la revendication 2, dans laquelle ladite composition de caoutchouc de silicone liquide comprend 1) un diorgano-polysiloxane contenant au moins deux radicaux alcényles par molécule, un organohydrogénopolysiloxane contenant au moins deux atomes d'hydrogène liés au silicium par molécule et ledit catalyseur d'hydrosilation, ou 2) un diorganopolysiloxane terminalement hydroxylé, ledit organohydrogéno-polysiloxane et un composé organique de l'étain en tant que catalyseur de durcissement.

**4.** Composition selon la revendication 3, dans laquelle les radicaux hydrocarbonés liés au silicium autres que lesdits radicaux alcényles présents dans ledit diorganopolysiloxane et ledit organohydrogéno-polysiloxane sont des radicaux méthyle.

**5.** Composition selon la revendication 1, dans laquelle lesdites particules sont dispersées dans une peinture.

**6.** Composition selon la revendication 1, dans laquelle lesdites particules sont dispersées dans un caoutchouc.

**7.** Composition selon la revendication 1, dans laquelle lesdites particules sont dispersées dans un cosmétique en poudre.